# EUROPEAN PATENT APPLICATION

(11) **EP 4 843 566 A1**
(43) Date of publication of application: **25.11.2026**
(21) Application number: 26179406.9
(22) Date of filing: 18.05.2026
(51) Int. Cl.: A61K 9/20

(54) **PHARMACEUTICAL COMPOSITION COMPRISING IVACAFTOR**

(30) Priority: 19.05.2025 EP 25386021
(71) Applicant: PharmaPath S.A., 12351 Agia Varvara (GR)
(72) Inventor: KONTOPANOU, Evanthia, 14561 Kifisia (GR); VOUVALIDIS, Ioannis, 14452 Metamorfosi (GR)
(74) Representative: Roukounas, Dimitrios

(57) **Abstract**

A granule comprising amorphous ivacaftor, or a pharmaceutically acceptable salt thereof. The granule comprises a solid substrate, which comprises a mixture of lactose monohydrate, croscarmellose sodium and microcrystalline cellulose, and a solid dispersion layered on the solid substrate, wherein the solid dispersion comprises ivacaftor, or a pharmaceutically acceptable salt thereof, hydroxypropyl methylcellulose acetate succinate and sodium lauryl sulfate. The granule is produced by fluid-bed granulation.

## Description

### FIELD OFTHE INVENTION

The present invention relates to solid pharmaceutical compositions comprising ivacaftor, or a pharmaceutically acceptable salt thereof.

### BACKGROUND OF THE INVENTION

Ivacaftor is chemically known as N-(2,4 di-tert-butyl-5-hydroxyphenyl)-I,4-dihydro-4-oxoquinoline-3-carboxamide and has the following structural formula:

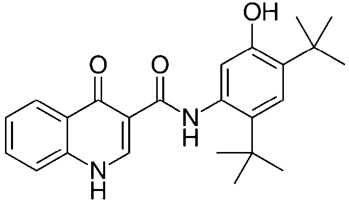

Ivacaftor is a potent and selective Cystic Fibrosis Transmembrane Regulator (CFTR) potentiator used in the treatment of cystic fibrosis with a certain mutation in CFTR gene.

Medicinal products comprising ivacaftor are commercially available as tablets and granules for oral administration under the brand name KALYDECO^{®}.

U.S. Patent No. 7,495,103 discloses modulators of ATP-binding cassette transporters such as ivacaftor and also discloses methods of treating CFTR transporter mediated diseases using such modulators.

U.S. Patent No. 7,553,855 discloses a pharmaceutical composition comprising: N-(5-hydroxy 2,4-ditert-butyl-phenyl)-4-oxo-lH-quinoline-3-carboxamide (ivacaftor), PEG 400, and PVP K30.

U.S. Patent No. 8,410,274 discloses a solid dispersion of amorphous N- [2,4-bis (I, I dimethylethyl)-5-hydroxyphenyl]-I,4-dihydro-4-oxoquinoline-3-carboxamide and pharmaceutical compositions comprising the solid dispersion.

U.S. Patent No. 8,163,772 discloses solid forms of ivacaftor in form of co-forms with 2-methyl butyric acid, propylene glycol, PEG400.KOAc, lactic acid, isobutyric acid, propionic acid, ethanol, 2-propanol, water, besylate, hemibesylate, and besylate monohydrate

U.S. Patent No. 8,883,206 discloses a pharmaceutical composition of ivacaftor in form of solid dispersion with hydroxypropyl methylcellulose acetate succinate (HPMCAS) along with other excipients.

U.S. Patent Publication No. 2015/0010628 discloses a pharmaceutical composition comprising a solid dispersion of amorphous or substantially amorphous ivacaftor, a filler, a sweetener, a disintegrant, a glidant and a lubricant, and optionally a wetting agent.

Ivacaftor is a white to off-white powder. It is freely soluble in methyl ethyl ketone/water mixture, soluble in 2-methyl tetrahydrofuran and PEG 400, slightly soluble in methanol, acetone and ethanol and practically insoluble in water (<0.05 microgram/mL) and buffers with pH 1.0 7.0. Ivacaftor has been isolated in an amorphous form and in crystalline forms. Various crystalline polymorphic forms are known and Form C is the most thermodynamically stable form. The absorption of ivacaftor in mouse, rats, rabbits and dogs is rapid, following oral administration of aqueous suspensions of the amorphous form, and bioavailability ranges from 30 to 100%. At the same time the crystalline form in suspension has a low oral bioavailability.

Due to low solubility and low bioavailability of crystalline form of ivacaftor, the marketed KALYDECO^{®} tablet is prepared in two stages, namely, manufacture of spray dispersion and tabletting. The spray-dried dispersion (SDD) is manufactured using solvent based spray-drying followed by secondary drying to remove residual solvents. In the second stage, the amorphous SDD is blended with additional excipients, compressed into a core tablet, film-coated and printed to form the final drug product. Due to ivacaftor being insoluble in water, it needs to be spray-dried with excipients to produce an amorphous form of the active substance, which circumvents the solubility limited bioavailability issues of the crystalline form and hence is better absorbed. The formulation and production process of KALYDECO^{®} is disclosed in EP3345625 A1.

KALYDECO^{®} comprises about 34.1 wt % of a solid dispersion by weight of the composition:
- wherein the dispersion comprises about 80 wt % of substantially amorphous Ivacaftor by weight of the dispersion,
- about 19.5 wt % of HPMCAS by weight of the dispersion,
- and about 0.5 wt % SLS by weight of the dispersion;
- about 30.5 wt % of microcrystalline cellulose by weight of the composition;
- about 30.4 wt % of lactose by weight of the composition;
- about 3 wt % of Croscarmellose sodium by weight of the composition;
- about 0.5 wt % of SLS by weight of the composition;
- about 0.5 wt % of colloidal silicon dioxide by weight of the composition;
- and about 1 wt % of magnesium Stearate by weight of the composition.

Spray-drying is the most accepted solid dispersion technology to achieve amorphous solid dispersion of API in drug products.

Fluid-bed granulation is another process that can be used for the preparation of amorphous solid dispersions. According to this process, a solution of the active pharmaceutical ingredient and a polymer is prepared in a suitable solvent. The solvent is then sprayed onto a solid substrate. Then the solvent evaporates and layering of the amorphous solid dispersion on the solid substrate takes place.

However, fluid-bed granulation can be challenging for the production of amorphous solid dispersions due to stickiness of the material and over agglomeration. Furthermore, due to the fact that the solution of the active pharmaceutical ingredient and the polymer is sprayed on the solid substrate, e.g. a dry mix, the formation of a solid dispersion in which the active pharmaceutical ingredient is in amorphouls form may not occur. Thus, in the production of amorphous solid dispersions of poorly soluble active pharmaceutical ingredients, the use of spray-drying is preferred over the use of fluid-bed granulation.

### SUMMARY OF INVENTION:

The present invention provides a granule comprising
a) a solid substate comprising a mixture of lactose monohydrate, croscarmellose sodium and microcrystalline cellulose, and
b) a solid dispersion layered on the solid substate, wherein the solid dispersion comprises ivacaftor, or a pharmaceutically acceptable salt thereof, hydroxypropyl methylcellulose acetate succinate and sodium lauryl sulfate,
wherein the granule is produced by fluid-bed granulation and wherein at least 90% of ivacaftor or a pharmaceutically acceptable salt thereof in the granule is in amorphous form.

The granule of the present invention exhibits high stability of the active ingredient. Furthermore, the granule exhibits high dissolution of the active ingredient.

The present invention also provides a pharmaceutical composition comprising the granule of the present invention.

The present invention further provides a unit dosage form comprising the granule or the composition of the present invention.

### DETAILED DESCRIPTION OF THE INVENTION

The present inventors have found that a solid dispersion of ivacaftor, or a pharmaceutically acceptable salt thereof, wherein at least 90% of ivacaftor, or its salt, is amorphous, can be formed by fluid-bed granulation on a solid substrate comprising lactose monohydrate, croscarmellose sodium and microcrystalline cellulose.

Thus, the present invention provides a granule comprising
a) a solid substrate comprising a mixture of lactose monohydrate, croscarmellose sodium and microcrystalline cellulose, and
b) a solid dispersion layered on the solid substrate, wherein the solid dispersion comprises ivacaftor, or a pharmaceutically acceptable salt thereof, hydroxypropyl methylcellulose acetate succinate and sodium lauryl sulfate,
wherein the granule is produced by fluid-bed granulation and wherein at least 90% of ivacaftor or a pharmaceutically acceptable salt thereof in the granule is in amorphous form.

Preferably, at least 95% of ivacaftor, or a pharmaceutically acceptable salt thereof in the granule is in amorphous form. More preferably, al least 99% of ivacaftor, or a pharmaceutically acceptable salt thereof in the granule is in amorphous form.

Preferably, the granule comprises from 25 wt % (weight percent) to 31 wt % ivacaftor, or a pharmaceutically acceptable salt thereof. More preferably, the granule comprises from 27 wt % to 29 wt % ivacaftor, or a pharmaceutically acceptable salt thereof.

Preferably, the granule comprises from 4 wt % to 14 wt % hydroxypropyl methylcellulose acetate succinate. More preferably. the granule comprises from 5 wt % to 13 wt % hydroxypropyl methylcellulose acetate succinate.

Preferably, the granule comprises from 0.05 wt % to 0.50 wt % sodium lauryl sulfate. More preferably. the granule comprises from 0.07 wt % to 0.30 wt % sodium lauryl sulfate.

Preferably, the granule comprises from 22 wt % to 35 wt % microcrystalline cellulose. More preferably. the granule comprises from 26 wt % to 31 wt % microcrystalline cellulose.

Preferably, the granule comprises from 25 wt % to 37 wt % lactose monohydrate. More preferably. the granule comprises from 28 wt % to 34 wt % lactose monohydrate.

The above wt % relate to g of an ingredient per 100g of granules.

According to a preferred embodiment, the solid substrate of the granule consists of a mixture of lactose monohydrate, croscarmellose sodium and microcrystalline cellulose and the solid dispersion consists of ivacaftor, or a pharmaceutically acceptable salt thereof, hydroxypropyl methylcellulose acetate succinate and sodium lauryl sulfate.

Ivacaftor can form a pharmaceutically acceptable acid addition salt with an inorganic or organic acid. Examples of acids that can be used to form a pharmaceutically acceptable salts of ivacaftor include hydrochloric acid, hydrobromic acid, sulfuric acid, phosphoric acid, oxalic acid, lactic acid, malic acid, maleic acid, malonic acid, tartaric acid, succinic acid, fumaric acid, acetic acid, citric acid, 4-hydroxy benzoic acid, 2,5-dihydroxy benzoic acid, adipic acid, methansulphonic acid, benzesulphonic acid and p- toluenesulphonic acid.

Preferably, the granule of the present invention comprises ivacaftor.

The granule of the present invention is produced by fluid-bed granulation. Preferably, the process for the production of the granule comprises
a) preparing a solution of hydroxypropyl methylcellulose acetate succinate, sodium lauryl sulfate and ivacaftor, or a pharmaceutically acceptable salt therefore, in a solvent,
b) dry mixing lactose monohydrate, croscarmellose sodium and microcrystalline cellulose, thereby obtaining a solid substrate,
c) spraying in a fluid-bed granulator the solution obtained in step a) on the solid substrate obtained in step b) and evaporating the solvent to obtain the granule.

Preferably, the solvent in the above process is a mixture of 91 wt % methyl ethyl ketone and 9 wt % purified water.

The present invention provides also a pharmaceutical composition comprising the granule of the present invention.

Preferably, the composition comprises at least 90 wt % granules of the present invention.

The pharmaceutical composition may comprise one or more extragranular excipients. Examples of extragranular excipients that can be used in the pharmaceutical composition of the present invention include disintegrants, glidants and lubricants.

Examples of disintegrants include croscarmelose sodium, carboxymethylcellulose calcium, carboxymethylcellulose sodium, crospovidone, cellulose, sodium starch glycolate, starch and pregelatinized starch.

Examples of glidants include colloidal silicon dioxide, calcium silicate, and talc.

Examples of lubricants include magnesium stearate, sodium lauryl sulphate and stearic acid.

According to a preferred embodiment, the extragranular excipients are croscarmellose sodium, sodium lauryl sulfate, colloidal silicon dioxide and magnesium stearate.

Preferably, the pharmaceutical composition comprises at least 90 wt % granules as defined above, from 1 wt % to 3 wt % extragranular croscarmellose sodium, from 0.40 wt % to 0.60 wt % extragranular sodium lauryl sulfate, from 0.40 wt % to 0.60 wt % extragranular colloidal silicon dioxide and from 0.80 wt % to 1.20 wt % extragranular magnesium stearate. The above wt % relate to g of an ingredient, or of granules, per 100 g of the composition.

The present invention provides also a unit dosage form comprising the granule or the composition of the present invention. Examples of unit dosage forms include a sachet, a tablet and a capsule. When the unit dosage form is a sachet, it may contain the granules of the present invention without any extragranular excipients, or with extragranular excipients. Preferably, the unit dosage form is a tablet.

When the unit dosage form is a tablet, it preferably exhibits dissolution of ivacaftor, or a pharmaceutically acceptable salt thereof, of at least 80% in 30 minutes. Dissolution is measured with a standard USP Type II apparatus that employs a dissolution media of 0.4% sodium lauryl sulfate dissolved in 900 mL of 50 mM Sodium Phosphate Buffer (or a volume of media having the same ratio of SLS to 50 mM Sodium Phosphate Buffer) at a temperature of about 37 °C. A single experimental tablet is tested in each test vessel of the apparatus.

When the unit dosage form is a tablet, it preferably has a hardness of at least 50N.

When the unit dosage form is a tablet, it may be coated.

The granule (and the composition) of the present invention contains high percentage of amorphous ivacaftor, or a pharmaceutically acceptable salt thereof. This means that the granule (and the composition) of the present invention exhibits high bioavailability of the active ingredient. In addition, the granule (and the composition) of the present invention exhibits excellent stability of the active ingredient.

Moreover, the granule of the present invention is produced according to a process which is more convenient than spray-drying, by enabling the production of the granule in one step, instead of the spray-drying processes for the production of the ivacaftor compositions of the prior art. Namely, in the prior art processes, the amorphous solid dispersion of ivacaftor is produced first and then it is combined with excipients to form a composition that can be used to prepare a unit dosage form, such as a tablet. On the other hand, the granule of the present invention can be used as such in a unit dosage form, for example, in a sachet, without using extragranular excipients.

Furthermore, fluid-bed granulation requires lower operating temperatures than spray drying, which means that the amorphous solid dispersion as well as the active ingredient are better protected against degradation due to high temperatures.

In addition, given that the granule of the present invention already contains excipients (lactose monohydrate, croscarmellose sodium and microcrystalline cellulose) the amount of extragranular excipients in the composition of the present invention is lower than the amount of excipients of the ivacaftor compositions of the prior art. This means that the composition of the present invention exhibits better uniformity compared to the ivacaftor compositions of the prior art.

Furthermore, a tablet according to the present invention is bioequivalent to the commercially available KALYDECO^{®} tablets.

### EXAMPLES

### Example 1

A solvent system of methyl ethyl ketone (MEK) and purified water, formulated according to the ratio of 91 wt % MEK and 9 wt % purified water into this solvent system, Hypromellose acetate succinate (HPMCAS) (HG grade), SLS and ivacaftor were added according to the ratio 12.12 wt % hypromellose acetate succinate / 0.19 wt % SLS / 28.63 wt % ivacaftor, based on the total intragranular component. The actual amounts of ingredients used to generate this mixture are recited in Table 1 (the solvents are not shown in the table, since they are removed during fluid-bed granulation).

API polymer solution sprayed on dry mix of lactose monohydrate, croscarmellose sodium and microcrystalline cellulose. A fluid bed processor was used for granulation and drying. Dried granules contained less than 5% water content.

The colloidal silicon dioxide, croscarmellose sodium, sodium lauryl sulfate, lactose monohydrate and the microcrystalline cellulose were passed through a 30-mesh screen. The dried granules also passed through a 40-mesh screen.

**Table 1: Formulation Composition**

| **Ingredient** | **Amount (mg)** | **% w/w** |
|---|---|---|
| **Intragranular part** | | |
| Ivacaftor | 150.00 | 27.27 |
| Sodium lauryl sulfate | 0.98 | 0.18 |
| Hypromellose acetate succinate | 63.50 | 11.55 |
| Microcrystalline cellulose | 138.72 | 25.22 |
| Lactose monohydrate | 148.30 | 26.96 |
| Croscarmellose Sodium | 22.50 | 4.09 |

| **Extragranular part** | | |
|---|---|---|
| Colloidal silicon dioxide | 2.75 | 0.50 |
| Croscarmellose sodium | 15.00 | 2.73 |
| Sodium lauryl sulfate | 2.75 | 0.50 |
| Magnesium stearate | 5.50 | 1.00 |
| Total weight | **550.0** | **100.00** |

The screened excipients were loaded into a blender and blended with dried granules. The magnesium stearate was filtered through a 40-mesh screen sieve into the blending container and mixed. The resulting mixture was compressed into tablets having an initial target hardness of about not less than 50 N. A batch of capsule-shaped tablets from above was spray-coated with complete film coating system Blue to a weight gain of about 3.0 wt % using a coating pan.

### Example 2

Another exemplary ivacaftor tablet containing 150 mg of ivacaftor.

A batch of capsule-shaped tablets is formulated to have about 150 mg of ivacaftor per tablet using the amounts of ingredients recited in Table 2.

A solvent system of MEK and purified water was prepared according to the ratio 91 wt % MEK / 9 wt % purified water. Into this solvent system, Hypromellose acetate succinate polymer (HPMCAS) (HG grade), sodium lauryl sulfate, and ivacaftor were added according to the ratio 5.98 wt % hypromellose acetate succinate / 0.09 wt % SLS / 28.49 wt % Ivacaftor, based on the total intragranular component. The actual amounts of ingredients used to generate this mixture are recited in Table 2 (the solvents are not shown in the table, since they are removed during fluid-bed granulation).

API polymer solution sprayed on dry mix of lactose monohydrate, croscarmellose sodium and microcrystalline cellulose. A fluid bed processor was used for granulation and drying. Dried granules contained less than 5 % water content.

The colloidal silicon dioxide, croscarmellose sodium, sodium lauryl sulfate, lactose monohydrate and the microcrystalline cellulose were passed through a 30-mesh screen. The dried granules also passed through a 40-mesh screen.

**Table 2: Formulation Composition**

| **Ingredient** | **Amount (mg)** | % **w/w** |
|---|---|---|
| **Intragranular part** | | |
| Ivacaftor | 150.00 | 27.27 |
| Sodium lauryl sulfate | 0.48 | 0.09 |
| Hypromellose acetate succinate | 31.50 | 5.73 |
| Microcrystalline cellulose | 162.72 | 29.59 |
| Lactose monohydrate | 161.80 | 29.42 |
| Croscarmellose Sodium | **20.00** | 3.64 |

| **Extragranular part** | | |
|---|---|---|
| Colloidal silicon dioxide | 2.75 | 0.50 |
| Croscarmellose sodium | **12.50** | 2.27 |
| Sodium lauryl sulfate | 2.75 | 0.50 |
| Magnesium stearate | 5.50 | 1.00 |
| Total weight | **550.0** | **100.00** |

The screened excipients were loaded into a blender and blended with dried granules. The magnesium stearate was filtered through a 40-mesh screen sieve into the blending container and mixed. The resulting mixture was compressed into tablets having an initial target hardness of about not less than 50 N. A batch of capsule-shaped tablets from above was spray-coated with complete film coating system Blue to a weight gain of about 3.0 wt % using a coating pan.

### Example 3

A batch of capsule-shaped tablets was formulated to have 150 mg of Ivacaftor per tablet using the amounts of ingredients recited in Table 3.

A solvent system of MEK and purified water was prepared according to the ratio 91 wt % MEK / 9 wt % purified water. Into this solvent system, hypromellose acetate succinate polymer (HPMCAS) (HG grade), sodium lauryl sulfate, and ivacaftor were added according to the ratio 6.97 wt % hypromellose acetate succinate / 0.19 wt % SLS / 28.63 wt % ivacaftor, based on the total intragranular component. The actual amounts of ingredients used to generate this mixture are recited in Table 3 (the solvents are not shown in the table, since they are removed during fluid-bed granulation).

API polymer solution sprayed on dry mix of lactose monohydrate, croscarmellose sodium and microcrystalline cellulose. A fluid bed processor was used for granulation and drying. Dried granules contained less than 5 % water content.

The colloidal silicon dioxide, croscarmellose sodium, sodium lauryl sulfate, lactose monohydrate and the microcrystalline cellulose were passed through a 30-mesh screen. The dried granules also passed through a 40-mesh screen.

**Table 3: Formulation Composition**

| **Ingredient** | **Amount (mg)** | **% w/w** |
|---|---|---|
| **Intragranular part** | | |
| Ivacaftor | 150.00 | 27.27 |
| Sodium lauryl sulfate | 0.98 | 0.18 |
| Hypromellose acetate succinate | 36.50 | 6.64 |
| Microcrystalline cellulose | 152.22 | 27.68 |
| Lactose monohydrate | 161.80 | 29.42 |
| Croscarmellose Sodium | **22.50** | 4.09 |

| **Extragranular part** | | |
|---|---|---|
| Colloidal silicon dioxide | 2.75 | 0.50 |
| Croscarmellose sodium | **15.00** | 2.73 |
| Sodium lauryl sulfate | 2.75 | 0.50 |
| Magnesium stearate | 5.50 | 1.00 |
| Total weight | **550.0** | **100.00** |

The screened excipients were loaded into a blender and blended with dried granules. The magnesium stearate was filtered through a 40-mesh screen sieve into the blending container and mixed. The resulting mixture was compressed into tablets having an initial target hardness of about not less than 50 N. A batch of capsule-shaped tablets from above was spray-coated with complete film coating system Blue to a weight gain of about 3.0 wt % using a coating pan.

### Example 4

A batch of capsule-shaped tablets is formulated to have 150 mg of Ivacaftor per tablet using the amounts of ingredients recited in Table 4.

A solvent system of MEK and purified water was prepared according to the ratio 91 wt % MEK / 9 wt % purified water. Into this solvent system, hypromellose acetate succinate polymer (HPMCAS) (HG grade), sodium lauryl sulfate, and ivacaftor were added according to the ratio 7.96 wt % hypromellose acetate succinate / 0.28 wt % SLS / 28.76 wt % ivacaftor, based on the total intragranular component. The actual amounts of ingredients used to generate this mixture are recited in Table 4 (the solvents are not shown in the table, since they are removed during fluid-bed granulation).

API polymer solution sprayed on dry mix of lactose monohydrate, croscarmellose sodium and microcrystalline cellulose. A fluid bed processor was used for granulation and drying. Dried granules contained less than 5 % water content.

The colloidal silicon dioxide, croscarmellose sodium, sodium lauryl sulfate, lactose monohydrate and the microcrystalline cellulose were passed through a 30-mesh screen. The dried granules also passed through a 40-mesh screen.

**Table 4: Formulation Composition**

| **Ingredient** | **Amount (mg)** | % **w/w** |
|---|---|---|
| **Intragranular part** | | |
| Ivacaftor | 150.00 | 27.27 |
| Sodium lauryl sulfate | 1.48 | 0.27 |
| Hypromellose acetate succinate | 41.50 | 7.55 |
| Microcrystalline cellulose | 141.72 | 25.77 |
| Lactose monohydrate | 161.80 | 29.42 |
| Croscarmellose sodium | **25.00** | 4.545 |

| **Extragranular part** | | |
|---|---|---|
| Colloidal silicon dioxide | 2.75 | 0.50 |
| Croscarmellose sodium | **17.50** | 3.18 |
| Sodium lauryl sulfate | 2.75 | 0.50 |
| Magnesium stearate | 5.50 | 1.00 |
| Total weight | **550.0** | **100.00** |

The screened excipients were loaded into a blender and blended with dried granules. The magnesium stearate was filtered through a 40-mesh screen sieve into the blending container and mixed. The resulting mixture was compressed into tablets having an initial target hardness of about not less Than 50 N. A batch of capsule-shaped tablets from above was spray-coated with complete film coating system Blue to a weight gain of about 3.0 wt % using a coating pan.

### Example 5

A batch of capsule-shaped tablets was formulated without disintegrant to have 150 mg of ivacaftor per tablet using the amounts of ingredients recited in Table 5.

A solvent system of MEK and purified water was prepared according to the ratio 91 wt % MEK / 9 wt % purified water. Into this solvent system, hypromellose acetate succinate polymer (HPMCAS) (HG grade), sodium lauryl sulfate, and ivacaftor were added according to the ratio 6.77 wt % hypromellose acetate succinate / 0.18 wt % SLS / 27.83 wt % ivacaftor, based on the total intragranular component. The actual amounts of ingredients used to generate this mixture are recited in Table 5 (the solvents are not shown in the table, since they are removed during fluid-bed granulation).

API polymer solution sprayed on dry mix of lactose monohydrate and microcrystalline cellulose. A fluid bed processor was used for granulation and drying. Dried granules contained less than 5 % water content.

The colloidal silicon dioxide, sodium lauryl sulfate, lactose monohydrate and the microcrystalline cellulose were passed through a 30-mesh screen. The dried granules also passed through a 40-mesh screen.

**Table 5: Formulation Composition**

| **Ingredient** | **Amount (mg)** | % **w/w** |
|---|---|---|
| **Intragranular part** | | |
| Ivacaftor | 150.00 | 27.27 |
| Sodium lauryl sulfate | 0.98 | 0.18 |
| Hypromellose acetate succinate | 36.50 | 6.64 |
| Microcrystalline cellulose | 170.97 | 31.09 |
| Lactose monohydrate | 180.55 | 32.83 |

| **Extragranular part** | | |
|---|---|---|
| Colloidal silicon dioxide | 2.75 | 0.50 |
| Sodium lauryl sulfate | 2.75 | 0.50 |
| Magnesium stearate | 5.50 | 1.00 |
| Total weight | **550.0** | **100.00** |

The screened excipients were loaded into a blender and blended with dried granules. The magnesium stearate was filtered through a 40-mesh screen sieve into the blending container and mixed. The resulting mixture was compressed into tablets having an initial target hardness of about not less than 50 N. A batch of capsule-shaped tablets from above was spray-coated with complete film coating system Blue to a weight gain of about 3.0 wt % using a coating pan.

### Example 6

The tablets of Examples 1-5 were analysed for dissolution utilizing USP Dissolution apparatus II at a paddle speed of 65 RPM, volume 900 ml in 50 mM sodium phosphate buffer with 0.4% sodium dodecyl sulfate pH 6.8. They were analysed utilizing HPLC which sampled at 30 minutes' time points. The dissolution results are displayed in Table 6.

**Table 6: Dissolution results**

| **Time (minutes) & percent released** (NLT 85 % of labelled amount of Ivacaftor in 30 minutes) | | |
|---|---|---|
| **Sample** | **0 minutes** | **30 minutes** |
| Example 1 | 0% | 88 % |
| Example 2 | 0% | 93 % |
| Example 3 | 0% | 95 % |
| Example 4 | 0% | 92 % |
| Example 5 | 0% | 70.0 % |

| | | |
|---|---|---|
| NLT: Not less than. | | |

The dissolution results indicate that the tablet without the disintegrant (croscarmellose sodium) (Example 5) exhibits a slower release profile. In contrast, tablets containing the disintegrant in both intra and extragranular portions demonstrated faster release profiles, achieving more than 85% drug release within 30 minutes.

Furthermore, the dissolution results for Example 1 show that tablets containing a higher amount of hypromellose acetate succinate (63.50 mg per tablet) exhibit slightly lower dissolution compared to the tablets of Examples 2-4 containing lower amounts of the polymer.

### Stability results of Ivacaftor 150 mg film coated tablets:

The study evaluates the stability of film-coated tablets of Example 3 stored under different relative humidity (RH) and temperature conditions for up to 6 months. The parameters assessed include water content (% by Karl Fischer method / KF), impurities, and dissolution performance. The results are shown in Tables 7 and 8.

**Table 7: 40°C /75% RH**

| | **40°C/75% RH** | | | | | | |
|---|---|---|---|---|---|---|---|
| | **Initial** | **1 month** | | **3 months** | | **6 months** | |
| **Pack** | **PVC/ Aclar** | **PVC/ Aclar** | **Alu-Alu** | **PVC/ Aclar** | **Alu-Alu** | **PVC/ Aclar** | **Alu-Alu** |
| **Description** | Light blue, capsule shaped film coated tablets with score line on one side and plain on the other side. | | | | | | |
| **Assay** | 102.4 | 104.8 | 101.9 | 100.9 | 101.4 | 101.4 | 101.6 |
| **Water content (by KF)** | 3.76 | 4.3 | 4.2 | 4.12 | 4.3 | 4.56 | 4.31 |

| **Related substances** | | | | | | | |
|---|---|---|---|---|---|---|---|
| **Name of Impurity** | **% wt Imp** | **% wt Imp** | **% wt Imp** | **% wt Imp** | **% wt Imp** | **% wt Imp** | **% wt Imp** |
| Quinoline acid impurity | ND | ND | ND | ND | ND | ND | ND |
| Single Max. Unkn. Imp. | 0.07 | 0.05 | 0.05 | 0.05 | 0.05 | 0.08 | 0.07 |
| Total Impurities | **0.12** | **0.11** | **0.12** | **0.13** | **0.12** | **0.22** | **0.18** |

| **Dissolution:** | | | | | | | |
|---|---|---|---|---|---|---|---|
| Media:50 mM sodium phosphate buffer with 0.4% sodium dodecyl sulfate (SDS), pH 6.8: Speed: 65RPM; Apparatus: II (Paddle); Volume: 900 ml (NLT 85 % of labelled amount of ivacaftor in 30 minutes) | | | | | | | |
| **Time (minutes)** | **% drug release and %RSD** | | | | | | |
| 30 | 95.00 (0.99) | 93.00 (2.39) | 94.00 (0.82) | 97.00 (1.74) | 95.00 (1.64) | 92.00 (2.67) | 90.00 (1.44) |

| | | | | | | | |
|---|---|---|---|---|---|---|---|
| ND: Not detected. NLT: Not less than. | | | | | | | |

**Table 8: 25°C /60% RH and 30°C /65% RH**

| | **25°C /60% RH** | | | | **30°C /65% RH** | | | |
|---|---|---|---|---|---|---|---|---|
| | **3 months** | | **6 months** | | **3 months** | | **6 months** | |
| **Pack** | **PVC/ Aclar** | **Alu-Alu** | **PVC/ Aclar** | **Alu-Alu** | **PVC/ Aclar** | **Alu-Alu** | **PVC/ Aclar** | **Alu-Alu** |
| **Description** | Light blue, capsule shaped film coated tablets with score line on one side and plain on the other side. | | | | | | | |
| **Assay** | 101.5 | 101.2 | 101.9 | 102.0 | 100.8 | NP | 102.0 | 101.2 |
| **Water content (by KF)** | 4.10 | 4.3 | 4.41 | 4.38 | 4.12 | NP | 4.49 | 4.35 |

| **Related substances** | | | | | | | | |
|---|---|---|---|---|---|---|---|---|
| **Name of Impurity** | **% wt Imp** | **% wt Imp** | **% wt Imp** | **% wt Imp** | **% wt Imp** | **% wt Imp** | **% wt Imp** | **% wt Imp** |
| Quinoline acid impurity | ND | ND | ND | ND | ND | NP | ND | ND |
| Single Max. Unkn. Imp. | 0.04 | 0.03 | 0.04 | 0.03 | 0.04 | NP | 0.04 | 0.04 |
| Total Impurities | **0.07** | **0.07** | **0.10** | **0.09** | **0.08** | NP | **0.11** | **0.11** |
| **Dissolution:** | | | | | | | | |
| Media:50 mM Sodium Phosphate Buffer with 0.4% Sodium Dodecyl Sulfate (SDS), pH 6.8: Speed: 65RPM; Apparatus: II (Paddle); Volume: 900 ml (NLT 85 % of labelled amount of Ivacaftor in 30 minutes) | | | | | | | | |

| **Time (minutes)** | **% Drug release and %RSD** | | | | | | | |
|---|---|---|---|---|---|---|---|---|
| 30 | 96.0 (2.20) | 97.0 (1.61) | 92.0 (1.10) | 95.00 (3.05) | 94.0 (3.55) | NP | 94.0 (1.03) | 94.0 (1.94) |

| | | | | | | | | |
|---|---|---|---|---|---|---|---|---|
| ND: Not detected. NP: Not performed. NLT: Not less than. | | | | | | | | |

The stability study of film-coated tablets stored under 40°C/75% RH, 25°C/60% RH, and 30°C/65% RH for up to 6 months demonstrated acceptable results across all evaluated parameters. Water content showed a slight increase over time but remained within acceptable limits. Impurities, including single maximum and total related substances, remained within specified limits, with minor increases noted under 40°C/75% RH conditions. Dissolution profile in 0.05% Sodium Dodecyl Sulfate (SDS) in pH 6.8 Sodium Phosphate Buffer using Apparatus II (Paddle) at 50 RPM consistently met the criteria of NLT 85% release in 30 minutes across all conditions. These results confirm the tablets' stability and suitability for long term, intermediate and accelerated conditions.

The film-coated tablets exhibit excellent stability profiles under the tested conditions.

## Claims

1. A granule comprising
a) a solid substrate comprising a mixture of lactose monohydrate, croscarmellose sodium and microcrystalline cellulose, and
b) a solid dispersion layered on the solid substrate, wherein the solid dispersion comprises ivacaftor, or a pharmaceutically acceptable salt thereof, hydroxypropyl methylcellulose acetate succinate and sodium lauryl sulfate,
wherein the granule is produced by fluid-bed granulation and wherein at least 90% of ivacaftor, or a pharmaceutically acceptable salt thereof in the granule is in amorphous form.

2. The granule according to claim 1, wherein at least 95% of ivacaftor, or a pharmaceutically acceptable salt thereof in the granule is in amorphous form.

3. The granule according to claim 1 or 2, wherein the granule comprises from 25 wt % to 31 wt % ivacaftor, or a pharmaceutically acceptable salt thereof.

4. The granule according to any one of the preceding claims, wherein the granule comprises from 4 wt % to 14 wt % hydroxypropyl methylcellulose acetate succinate.

5. The granule according to any one of the preceding claims, wherein the granule comprises from 0.05 wt % to 0.50 wt % sodium lauryl sulfate.

6. The granule according to any one of the preceding claims, wherein the granule comprises from 22 wt % to 35 wt % microcrystalline cellulose.

7. The granule according to any one of the preceding claims, wherein the granule comprises from 25 wt % to 37 wt % lactose monohydrate.

8. The granule according to any one of the preceding claims, wherein the fluid-bed granulation comprises the following steps
a) preparing a solution of hydroxypropyl methylcellulose acetate succinate, sodium lauryl sulfate and ivacaftor, or a pharmaceutically acceptable salt therefore, in a solvent,
b) dry mixing lactose monohydrate, croscarmellose sodium and microcrystalline cellulose, thereby obtaining a solid substrate,
c) spraying in a fluid-bed granulator the solution obtained in step a) on the solid substrate obtained in step b) and evaporating the solvent to obtain the granule.

9. The granule according to any one of the preceding claims, wherein the solvent is a mixture of 91 wt % methyl ethyl ketone and 9 wt % purified water.

10. The granule according to any one of the preceding claims, wherein the solid substrate of the granule consists of a mixture of lactose monohydrate, croscarmellose sodium and microcrystalline cellulose and the solid dispersion consists of ivacaftor, or a pharmaceutically acceptable salt thereof, hydroxypropyl methylcellulose acetate succinate and sodium lauryl sulfate.

11. The granule according to any one of the preceding claims, wherein the granule comprises ivacaftor.

12. A pharmaceutical composition comprising the granule according to any one of claims 1 to 11 and a pharmaceutically acceptable extragranular excipient.

13. The pharmaceutical composition according to claim 12, comprising at least 90 wt % granules according to in any one of claims 1 to 11.

14. The pharmaceutical composition according to claim 12 or 13, wherein the composition comprises extragranular croscarmellose sodium, extragranular sodium lauryl sulfate, extragranular colloidal silicon dioxide and extragranular magnesium stearate.

15. The pharmaceutical composition according to any one of claims 12 to 14, wherein the composition comprises at least 90 wt % granules, from 1 wt % to 4 wt % extragranular croscarmellose sodium, from 0.40 wt % to 0.60 wt % extragranular sodium lauryl sulfate, from 0.40 wt % to 0.60 wt % extragranular colloidal silicon dioxide and from 0.80 wt % to 1.20 wt % extragranular magnesium stearate.
